# EUROPEAN PATENT APPLICATION

(11) **EP 4 553 150 A1**
(43) Date of publication of application: **14.05.2025**
(21) Application number: 23903767.4
(22) Date of filing: 08.11.2023
(51) Int. Cl.: C12N 5/077, C12N 5/0775, C12N 1/12, C12R 1/89

(54) **CELL CULTURE COMPOSITION CONTAINING MICROALGAE EXTRACT AND USE THEREOF**

(30) Priority: 15.12.2022 KR 20220175629
(71) Applicant: CJ CheilJedang Corporation, Seoul 04560 (KR)
(72) Inventor: SHIN, Won Sub, Seoul 04560 (KR); RYU, Ae Jin, Seoul 04560 (KR); KIM, Ji Young, Seoul 04560 (KR); JEON, Seungjib, Seoul 04560 (KR); JANG, Sunghoon, Seoul 04560 (KR); KANG, Hae-Won, Seoul 04560 (KR)
(74) Representative: Meissner Bolte Partnerschaft mbB
(86) International application number: PCT/KR2023/017908
(87) International publication number: WO 2024/128564

(57) **Abstract**

The present invention relates to a cell culture composition containing an extract from microalgae of the Thraustochytrid family. It has been found that when muscle cells are cultured in a medium containing an extract derived from the biomass of microalgae belonging to the Thraustochytrid family according to the present disclosure, the proliferative capacity of muscle cells is enhanced. Therefore, the cell culture composition including the microalgae extract can be used as a substitute for fetal bovine serum among the components of cell culture media.

## Description

### [TECHNICAL FIELD]

### Cross-Reference to Related Application(s)

The present application claims the priority based on Korean Patent Application No. 10-2022-0175629 filed on December 15, 2022, and the entire contents disclosed in the documents of the corresponding Korean Patent Application are incorporated by reference as a part of the present description.

The present invention relates to a composition for cell culture comprising a microalgal extract and uses thereof.

### [BACKGROUND ART]

Until now, fetal bovine serum (FBS) among basic culture medium components used for animal cell culture is the most popularly used component. However, ethic issues inevitably occur during a preparation process of fetal bovine serum, and various problems such as contamination issues and supply limitations and the like are scattered, so development of substitutes thereof is necessarily needed. Various studies have been progressed to develop a serum-free culture medium not using fetal bovine serum (KR 10-2021-0090560), but there is still a need to additionally add various kinds of hormones or growth factors in order to replace fetal bovine serum, so additional development is needed for user convenience.

In the present invention, an extract is obtained from biomass produced through fermentation of a Thraustochytrid-based microalgae, and is to be used as a culture medium component for cell culture. A microalgal biomass production and extraction process based on fermentation has advantages of being free to ethic issues unlike the conventional fetal bovine serum production process, and avoiding stability issues such as contamination issues and the like.

### [DETAILED DESCRIPTION OF THE INVENTION]

### [TECHNICAL PROBLEM]

The present invention provides a culture medium composition for cell culture, comprising a Thraustochytrid family microalgal extract.

Another embodiment provides a culture medium additive for cell culture, comprising a Thraustochytrid family microalgal extract.

Other embodiment provides a culture medium for cell culture, comprising the culture medium additive for cell culture.

Other embodiment provides a method for culturing cells using the culture medium composition for cell culture.

Other embodiment, provides a use of a Thraustochytrid family microalgal extract as a culture medium additive capable of promoting proliferation and survival of cells while replacing animal serum.

### [TECHNICAL SOLUTION]

As one aspect to achieve the above objects, the present invention relates to a culture medium composition for cell culture comprising a Thraustochytrid family microalgal extract.

As another aspect, the present invention relates to a culture medium additive for cell culture, comprising a Thraustochytrid family microalgal extract.

As other aspect, the present invention relates to a culture medium for cell culture, comprising the culture medium additive.

As other aspect, the present invention relates to a method for culturing cells, comprising culturing cells in the culture medium.

As other aspect, the present invention relates to a use of a Thraustochytrid family microalgal extract as a culture medium additive capable of promoting proliferation and survival of cells while replacing animal serum.

Hereinafter, the present invention will be described in more detail.

The present invention provides a culture medium composition for cell culture comprising a Thraustochytrid family microalgal extract.

In the present description, the term "cell culture" means a process of artificially growing living cells in vitro under an adjusted condition. In addition, it may grow and proliferate the cells by aseptically removing part of individual tissue, and smearing suspension released by degrading connecting materials between cells with an enzyme on the flat bottom of a culture dish such as a bottle or a petri dish.

The cell culture may comprise culturing cells to prepare cell cultured meat. Therefore, the composition may be a culture medium composition for preparing cultured meat. The culture medium composition of the present invention may comprise a Thraustochytrid family microalgal extract instead of commonly used fetal bovine serum. The Thraustochytrid family microalgal extract is a material rich in nutrients such as proteins and amino acids and the like, and can exclude stability problems, and can expect excellent productivity due to its low production cost compared to fetal bovine serum.

In the present description, the term "cultured meat" means edible meat to be obtained by collecting animal cells and then proliferating them by a cell engineering technology, and it can be said to be one field of cellular agriculture in which meat is obtained without passing through a process of raising livestock. In Korean, it is called cultured meat, meat analogue or artificial meat, and in English, it is also called in vitro meat as a meaning of growing in vitro, artificial meat as a meaning of synthesizing using stem cells by human, not natural, clean meat as a meaning of being produced in a clean production facility, not a traditional breeding facility, and a lab-grown meat, as it is made in a laboratory.

In the present description, the term "culture medium (culture media)" means a medium that enables to support growth, survival and differentiation of cells or stem cells in vitro, and includes all common media suitable for culture and differentiation of cells or stem cells used in the art. The type of the media and culture conditions may be selected at the technical level of the corresponding field depending on the type of cell. The medium used for culture is specifically a cell culture minimum culture medium (CCMM), and in general, it comprises carbon sources, nitrogen sources, and trace element components. The cell culture minimum medium may be for example, at least one selected from the group consisting of DMEM (Dulbecco's Modified Eagle's Culture medium), MEM (Minimal Essential Culture medium), BME (Basal Culture medium Eagle), RPMI 1640, F-10, F-12, DMEM/F12, α-MEM (α-Minimal Essential Culture medium), G-MEM (Glasgow's Minimal Essential Culture medium), IMDM (Iscove's Modified Dulbecco's Culture medium), MacCoy's 5A medium, AmnioMax complete medium, AminoMax ± medium, EBM (Endothelial Basal Culture medium) medium, Chang's Culture medium, MesenCult-XF, DMEM/HG (Dulbecco's Modified Eagle's Culture medium high glucose) medium, and MCDB+DMEM/LG (MCDB+Dulbecco's Modified Eagle's Culture medium low glucose) medium. In addition, the medium may comprise an antibiotic such as penicillin, streptomycin, gentamicin or a mixture of two or more thereof or the like. Therefore, the culture medium composition for cell culture of the present invention may comprise a Thraustochytrid family microalgal extract added to the cell culture minimum medium.

In the present description, the term "Thraustochytrid family microalgae" is heterotrophic microorganisms belonging to the order Thraustochytriales, and is known as oleaginous microorganisms capable of producing bio-oil containing unsaturated fatty acids such as DHA (docosahexaenoic acid), and is considered a good protein source as it is rich in proteins and amino acids.

The Thraustochytrid family microalgae may be any genus available to those skilled in the art. Specifically, it may be any one selected from the group consisting of the genus *Schizochytrium,* the genus *Aurantiochytrium,* the genus *Thraustochytrium* and the genus *UIkenia,* but not limited thereto.

In the present description, the term "extract" may mean resulting products such as liquid minutes obtained by immersing a target material in various solvents, and then extracting it in a room temperature or elevated temperature state for a certain period of time, and solids obtained by removing solvents from the liquid components and the like. Furthermore, in addition to the resulting products, it may be comprehensively interpreted to include extracted solutions themselves such as diluted solutions of the resulting products, concentrates thereof, crude purified materials, purified materials, or mixtures thereof, and the like, and extracts in all formulations which can be formed using extracted solutions. Moreover, the extract may include fractions obtained therefrom.

The extract may be extracted from a natural, hybrid or variant Thraustochytrid family microalgae of the Thraustochytrid family microalgae, and it may be extracted from Thraustochytrid family microalgal tissue culture.

The extraction solvent of the extract may be a protic polar solvent or non-protic polar and non-polar solvents. The protic polar solvent may be water, methanol, ethanol, propanol, isopropanol, or butanol. The non-protic polar solvent may be dichloromethane, tetrahydrofuran, ethyl acetate, acetonitrile, dimethylformamide, dimethyl sulfoxide, acetone, 2-butanone, or hexamethylphosphoramide. The non-polar solvent may be pentane, hexane, chloroform, or diethylether. The non-polar solvent excludes benzene. The solvent may be a C1-C6 alcohol, a C3-C10 ester, for example, a C3-C10 acetate, a C3-C10 ketone, a C1-C6 unsubstituted or halogenated hydrocarbon, a C2-C10 cyclic ether, a mixture thereof, or a mixture of at least one of the solvents and water. The solvent may be ethanol, propanol, acetonitrile, ethyl acetate, acetone, 2-butanone, chloroform, dichloromethane, hexane, a mixture thereof, or a mixture of at least one of the solvents and water. The hydrocarbon may be an alkane, alkene, or alkyne.

The extract may be extracted with at least one solvent selected from the group consisting of water; lower alcohols of C1 to C4 such as methanol, ethanol, propanol, butanol and the like; polyhydric alcohols such as glycerine, butylene glycol, propylene glycol and the like; and hydrocarbon solvents such as methyl acetate, ethyl acetate, acetone, benzene, hexane, diethyl ether, dichloromethane and the like. Specifically, the extract may be extracted with water as a solvent.

The Thraustochytrid family microalgal extract may be extracted as the Thraustochytrid family microalgal culture solution as it is, and may be extracted after being prepared as biomass by freeze-drying or dehydrating the culture solution with a centrifuge and then introducing other drying process using freeze dry, drum dry, spray dry, a granulator and the like, but not limited thereto.

In the present description, the term "biomass" may be a Thraustochytrid family microalgae itself, a culture thereof, a dry matter thereof, a lysate thereof, or a product produced by culturing or fermenting the microalgae, or it may be a concentrate or dry matter of the biomass, but not limited thereto.

The Thraustochytrid family microalgal extract may be used as Thraustochytrid family microalgae or Thraustochytrid family microalgal biomass is extracted by a common method, and for example, an extraction method such as hot water extraction, cold brew extraction, reduced pressure and high temperature extraction, boiling water extraction, room temperature extraction, fraction extraction, reflux cooling extraction, solvent extraction, steam distillation, ultrasonic extraction, eruption, compression, and the like, and preferably, it may be extracted using hot water extraction. In addition, if necessary, as the Thraustochytrid family microalgal extract, a commercially available Thraustochytrid family microalgal extract may be used.

The hot water extraction may be performed at 80 to 140°C, 85 to 135°C, 90 to 130°C, 95 to 125°C, 100 to 120°C, or 105 to 115°C, and in one specific embodiment, it may be performed at 110°C, but not limited thereto. In addition, the hot water extraction may be performed for 3 minutes or more, 5 minutes or more, 10 minutes or more, 1 to 30 minutes, 2 to 30 minutes, 3 to 30 minutes, 4 to 30 minutes, 5 to 30 minutes, 6 to 30 minutes, 7 to 30 minutes, 8 to 30 minutes, 9 to 30 minutes, 1 to 25 minutes, 2 to 25 minutes, 3 to 25 minutes, 4 to 25 minutes, 5 to 25 minutes, 6 to 25 minutes, 7 to 25 minutes, 8 to 25 minutes, 9 to 25 minutes, 1 to 20 minutes, 2 to 20 minutes, 3 to 20 minutes, 4 to 20 minutes, 5 to 20 minutes, 6 to 20 minutes, 7 to 20 minutes, 8 to 20 minutes, 9 to 20 minutes, 1 to 15 minutes, 2 to 15 minutes, 3 to 15 minutes, 4 to 15 minutes, 5 to 15 minutes, 6 to 15 minutes, 7 to 15 minutes, 8 to 15 minutes, or 9 to 15 minutes, and in one specific embodiment, it may be performed for 10 minutes, but not limited thereto.

The term "fractions" in the present description means resulting products obtained by performing fractionation in order to separate a specific component or a specific component group from a mixture comprising various components.

The fractionation method for obtaining the fractions is not particularly limited, and may be performed according to a commonly used method in the art. Non-restrictive examples of the fractionation method, include a fractionation method performed by treating various solvents, an ultrafiltration fractionation method performed by passing an ultrafiltration membrane with a certain molecular weight cut-off value, a chromatography fractionation method performing a variety of chromatography (produced for separation depending on size, charge, hydrophobicity or affinity), and combinations thereof, and the like.

The type of fractionation solvent used for obtaining the fractions is not particularly limited, and any solvent known in the art may be used. Non-restrictive examples of the fractionation solvent may include polar solvents such as water, alcohols having 1 to 4 carbon atoms and the like; non-polar solvents such as hexane, ethyl acetate, chloroform, dichloromethane and the like; or mixed solvents thereof. They may be used alone or used in combination of one or more, but are not limited thereto.

The Thraustochytrid family microalgal extract may be comprised at a concentration of 25 g/L or less, specifically, at a concentration of 23.6 g/L or less, in the culture medium composition for cell culture. For example, the concentration may be 0.001 to 25 g/L, 0.005 to 25 g/L, 0.01 to 25 g/L, 0.05 to 25 g/L, 0.1 to 25 g/L, 0.2 to 25 g/L, 0.4 to 25 g/L, 0.8 to 25 g/L, 1 to 25 g/L, 2 to 25 g/L, 3 to 25 g/L, 0.001 to 23.6 g/L, 0.005 to 23.6 g/L, 0.01 to 23.6 g/L, 0.05 to 23.6 g/L, 0.1 to 23.6 g/L, 0.2 to 23.6 g/L, 0.4 to 23.6 g/L, 0.8 to 23.6 g/L, 1 to 23.6 g/L, 2 to 23.6 g/L, 3 to 23.6 g/L, 0.001 to 22 g/L, 0.005 to 22 g/L, 0.01 to 22 g/L, 0.05 to 22 g/L, 0.1 to 22 g/L, 0.2 to 22 g/L, 0.4 to 22 g/L, 0.8 to 22 g/L, 1 to 22 g/L, 2 to 22 g/L, 3 to 22 g/L, 0.001 to 20 g/L, 0.005 to 20 g/L, 0.01 to 20 g/L, 0.05 to 20 g/L, 0.1 to 20 g/L, 0.2 to 20 g/L, 0.4 to 20 g/L, 0.8 to 20 g/L, 1 to 20 g/L, 2 to 20 g/L, 3 to 20 g/L, 0.001 to 15 g/L, 0.005 to 15 g/L, 0.01 to 15 g/L, 0.05 to 15 g/L, 0.1 to 15 g/L, 0.2 to 15 g/L, 0.4 to 15 g/L, 0.8 to 15 g/L, 1 to 15 g/L, 2 to 15 g/L, 3 to 15 g/L, 0.001 to 12 g/L, 0.005 to 12 g/L, 0.01 to 12 g/L, 0.05 to 12 g/L, 0.1 to 12 g/L, 0.2 to 12 g/L, 0.4 to 12 g/L, 0.8 to 12 g/L, 1 to 12 g/L, 2 to 12 g/L, 3 to 12 g/L, 0.001 to 11 g/L, 0.005 to 11 g/L, 0.01 to 11 g/L, 0.05 to 11 g/L, 0.1 to 11 g/L, 0.2 to 11 g/L, 0.4 to 11 g/L, 0.8 to 11 g/L, 1 to 11 g/L, 2 to 11 g/L, 3 to 11 g/L, 0.001 to 10 g/L, 0.005 to 10 g/L, 0.01 to 10 g/L, 0.05 to 10 g/L, 0.1 to 10 g/L, 0.2 to 10 g/L, 0.4 to 10 g/L, 0.8 to 10 g/L, 1 to 10 g/L, 2 to 10 g/L, 3 to 10 g/L, 0.001 to 8 g/L, 0.005 to 8 g/L, 0.01 to 8 g/L, 0.05 to 8 g/L, 0.1 to 8 g/L, 0.2 to 8 g/L, 0.4 to 8 g/L, 0.8 to 8 g/L, 1 to 8 g/L, 2 to 8 g/L, 3 to 8 g/L, 0.001 to 5 g/L, 0.005 to 5 g/L, 0.01 to 5 g/L, 0.05 to 5 g/L, 0.1 to 5 g/L, 0.2 to 5 g/L, 0.4 to 5 g/L, 0.8 to 5 g/L, 1 to 5 g/L, 2 to 5 g/L, 3 to 5 g/L, 0.001 to 4 g/L, 0.005 to 4 g/L, 0.01 to 4 g/L, 0.05 to 4 g/L, 0.1 to 4 g/L, 0.2 to 4 g/L, 0.4 to 4 g/L, 0.8 to 4 g/L, 1 to 4 g/L, 2 to 4 g/L, or 3 to 4 g/L, and specifically, it may be 0.8 to 4 g/L.

The cell may be a somatic cell, a germ cell, a stem cell, a cancer cell, a cell line, a cultured cell (in vitro), a graft cell, and a primary cultured cell (in vitro and ex vivo), and an in vivo cell, a cell of a eukaryotic organism or a mammalian cell including humans, and for example, the cell may be selected from the group consisting of cancer cells, stem cells, vascular endothelial cells, white blood cells, immune cells, epithelial cells, germ cells, fibroblasts, muscle cells, bone marrow cells, epidermal cells, osteoblasts, and nerve cells. In addition, the eukaryotic organism may be an insect, amphibian, bird (chicken, turkey, duck, etc.) or mammal (primate such as human, monkey, and the like, dog, pig, cow, sheep, goat, mouse, rat, etc.).

The cell may be a cell isolated from an individual or a eukaryotic organism.

The cell may be an undifferentiated cell, cell in differentiation, or cell which has completed differentiation, and specifically, it may be a stem cell.

In the present description, the term "stem cell" means a cell having potency and self-renewal ability. The stem cell is divided into pluripotency, multipotency or unipotency according to the differentiation ability. The stem cell may be at least one selected from the group consisting of an embryonic stem cell (ESC) (embryonic inner cell before implantation), an adult stem cell (undifferentiated cell present in each tissue and organ), and an induced pluripotent stem cell (iPSC) (cell in which dedifferentiation is induced by inserting a gene and/or a protein to a somatic cell, or induced pluripotent stem cell).

The type and origin of the stem cell are not limited as long as it has potency and self-renewal ability. The stem cell, may be derived from, for example, a mammal, human, monkey, pig, horse, cow, sheep, dog, cat, mouse or rabbit. The stem cell may be derived from isolated umbilical cord, placenta, fat, bone marrow, muscle, umbilical cord blood, or amniotic fluid. The term "isolated" means existing in an environment different from an environment of cells or tissue. Isolating umbilical cord, placenta, fat, bone marrow, muscle, umbilical cord blood, or amniotic fluid, and obtaining stem cells may be performed by a conventional anatomical method and a known method.

The cell may be a muscle cell or myogenic stem cell, and specifically, may be derived from cattle such as chicken, bovine and porcine and the like, and more specifically, may be a muscle cell or myogenic stem cell derived from bovine or porcine.

In the present description, the term "myogenic stem cell" means a cell having characteristics of myogenic stem cells including all of proliferation without transformation, unlimited proliferation, self-reproduction ability and ability to differentiate into muscle, and any cell showing self-reproduction ability or unlimited proliferation ability and muscle differentiation ability may be included without limitation. The self-reproduction ability and muscle differentiation ability can be confirmed with markers. The myogenic stem cells may be interchangeably used with satellite cells (SCs).

According to one example, it has been confirmed that the culture medium composition comprising the Thraustochytrid family microalgal extract shows significantly excellent cell proliferation efficacy in myogenic stem cells.

The composition may further comprise serum. The serum may be at least one selected from the group consisting of fetal bovine serum (FBS), bovine calf serum (BCS), human serum and horse serum (HS), and specifically, it may be fetal bovine serum.

The composition may comprise serum and a Thraustochytrid family microalgal extract, and the serum may be comprised at a weight ratio of 1000:1 to 1:1 based on the Thraustochytrid family microalgal extract (serum: Thraustochytrid family microalgal extract). For example, the weight ratio of the serum and chlorella extract may be 1000:1 to 1:1, 800:1 to 1:1, 600:1 to 1:1, 500:1 to 1:1, 400:1 to 1:1, 350:1 to 1:1, 300:1 to 1:1, 250:1 to 1:1, 200:1 to 1:1, 150:1 to 1:1, 100:1 to 1:1, 50:1 to 1:1, 40:1 to 1:1, 30:1 to 1:1, 25:1 to 1:1, 20:1 to 1:1, 15:1 to 1:1, 12.5:1 to 1:1, 10:1 to 1:1, 7.5:1 to 1:1, 5:1 to 1:1 or 2.5:1 to 1:1.

The culture composition allows subculture of cells.

In the present description, the term, "passage" means replacing a culture container, or culturing by dividing cell groups, in a method of culturing by continuing generations of cells, in order to culture cells, specifically, stem cells, in a healthy state, continuously for a long period of time. Replacing a culture container once or culturing by dividing cell groups is called 1 passage. In the present invention, the passage can be interchangeably used with generation.

The culture may be growth and proliferation. In the present description, the term "growth and proliferation" means an increase in the number of cells. The culture may be undifferentiated proliferation. The undifferentiated proliferation means proliferating stem cells into cells having the same properties as the original cells, that is, potency and self-renewal, without being differentiated into specific cells. The term "differentiation" means a phenomenon in which cells become specialized in structure or function while they divide and proliferate and grow, that is, changing in the form or function of cells, tissue, and the like of living organisms to perform a given task to each. Measuring or determining the degree of differentiation into a specific cell type may be performed by a method well known in the art. In addition, the differentiation may be confirmed by investigating a cell form using an optical microscope or confocal microscope, while measuring a cell surface marker (for example, staining cells with a tissue-specific or cell-label specific antibody) and a change in cell form (for example, nuclear ratio/cytoplasmic ratio) using a method such as flow cytometry or immunocytochemistry, or measuring a change in gene expression using a well-known method in the art such as polymerase chain reaction (PCR) and gene-expression profiles.

In addition, the present invention provides a culture medium additive for cell culture, comprising a Thraustochytrid family microalgal extract.

The same parts as the contents described above are applied to the method in deed.

The culture medium additive may replace all or part of animal serum comprised in a culture medium, so the culture medium for cell culture containing the culture medium additive may have a reduced serum content than a general culture medium (containing about 10% serum). In addition, the low-serum culture medium comprising the culture medium additive may exhibit the cell growth rate, cell viability and subculture efficiency equivalent or significantly excellent to a culture medium containing 10% serum.

In addition, the present invention provides a method for cell culture, comprising culturing isolated cells using the culture medium composition for cell culture.

The same parts as the contents described above are applied to the method in deed.

The isolated cells may be cells isolated from livestock, and specifically, may be cells isolated from livestock such as cattle, pigs or chickens or the like.

The method for cell culture may be a method for culturing cells to prepare cultured meat, and specifically, may be proliferating and culturing myogenic stem cells to prepare cultured meat. Therefore, cultured meat may be prepared using the cells, specifically, myogenic stem cells cultured by the method.

The culture form may be common two-dimensional or three-dimensional culture known in the art. However, three-dimensional culture may be preferable to implement similar tissues to actual biological tissues by cell-to-cell interaction. Specific examples of the three-dimensional culture include a method for arranging cells in 3D porous scaffolds, scaffold-free platforms through cells themselves or a cell sheet technology, or microchips, a method using hydrogels, and a method using a bioreactor.

In addition, the present invention provides a use of the Thraustochytrid family microalgal extract as a culture medium additive capable of promoting proliferation and survival of cells while replacing animal serum.

In the use of the present application, the Thraustochytrid family microalgae, culture medium additive, and the like are as described above.

### [ADVANTAGEOUS EFFECTS]

It has been confirmed that the cell proliferation ability of bovine muscle cells is improved, when muscle cells are cultured in a culture medium comprising the extract extracted from biomass of microalgae belonging to the Thraustochytrid family of the present invention, and thus, the composition for cell culture comprising a microalgal extract can be used by replacing fetal bovine serum among culture medium components for cell culture. Furthermore, the microalgal extract comprised in the composition for cell culture of the present invention passes through a production process based on fermentation, so it is free from various ethical issues and contamination issues unlike the conventional production process of fetal bovine serum, and has an advantage in that continuous mass production is possible while maintaining a more consistent quality.

### [BRIEF DESCRIPTION OF THE DRAWINGS]

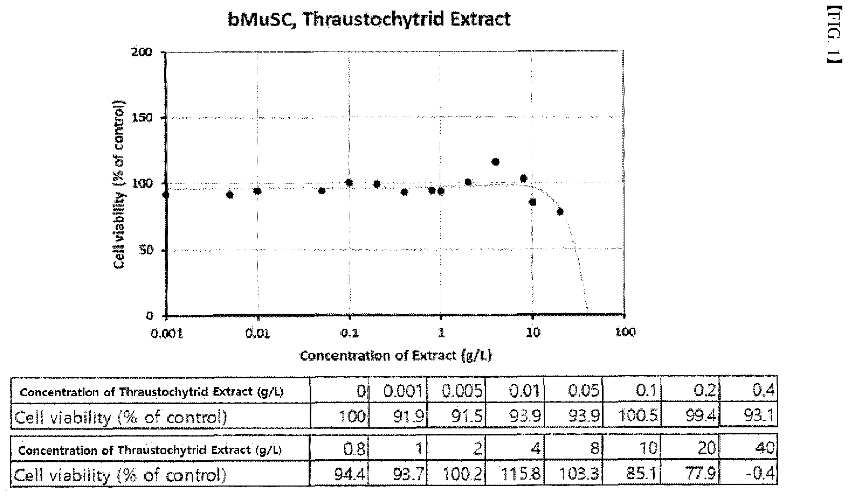

FIG. 1 is a diagram showing the cytotoxicity and cell proliferation effects of the Thraustochytrid family microalgal extract for bovine myogenic stem cells. They were expressed as mean (N=3) ± standard deviation.

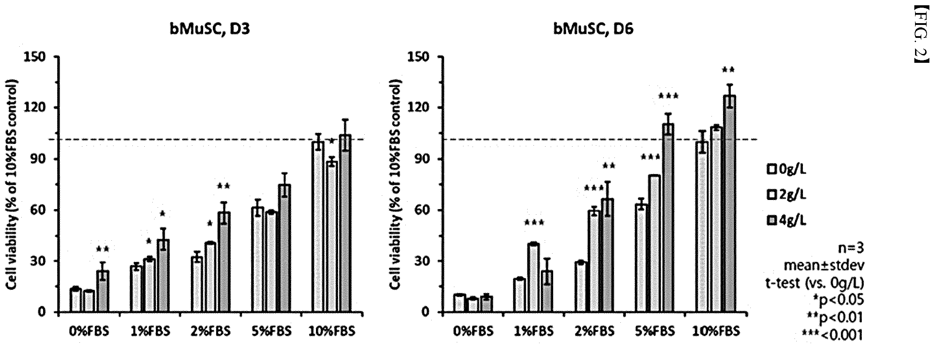

FIG. 2 is diagrams showing the cell proliferation effect of the culture solution comprising FBS and the Thraustochytrid family microalgal extract at various concentrations for bovine myogenic stem cells. It was expressed as mean (N=3) ± standard deviation, and statistical comparison was carried out between cells cultured with a culture medium comprising the Thraustochytrid family microalgal extract for cells cultured without the Thraustochytrid family microalgal extract for the same culture period. (* p<0.05, ** p<0.01, *** p<0.001, Student's T test)

### [MODE FOR INVENTION]

Hereinafter, the present invention will be described in more detail by examples. However, these examples are intended to illustratively describe at least one specific embodiment, and the scope of the present invention is not limited by these examples.

### Example 1. Obtainment of microalgal extract

A *Schizochytrium* sp. strain belonging to a Thraustochytrid family microalgae was under dark culture and then dried to secure biomass.

Specifically, the *Schizochytrium* sp. strain was under fermentation in a 5L fermenter containing a sterilized MJW01 culture medium (Glucose 30 g/L, MgSO₄·7H2O 3.0 g/L, Na₂SO₄ 10 g/L, NaCl 1.0 g/L, yeast extract 9.0 g/L, MSG·1H₂O 1.0 g/L, NaNO₃ 1.0 g/L, KH₂PO₄ 0.1 g/L, K₂HPO₄ 0.5 g/L, CaCl₂ 0.5 g/L, vitamin mixed solution 10 ml/L). The microbial cell culture solution was freeze-dried as the culture solution was, or freeze-dried after dehydrating by a centrifuge to secure biomass. The secured microalgal biomass was dispersed in purified water at a concentration of 1.5%, and then heated at 110°C for 10 minutes to progress hot water extraction. The extracted solution was filtered to remove remaining floating materials, and then it was freeze-dried to obtain a Thraustochytrid microalgal extract. The individual amino acid content and the total amino acid content based on dry weight of the obtained Thraustochytrid microalgal extract were measured and shown in Table 1 below.

**[Table 1]**

| Kind of amino acid | Amino acid content based on dry weight (%) |
|---|---|
| Asp | 3.19 |
| Thr | 1.17 |
| Ser | 1.10 |
| Glu | 24.58 |
| Gly | 1.59 |
| Ala | 1.75 |
| Cys | 0.31 |
| Val | 1.92 |
| Met | 0.57 |
| Ile | 1.13 |
| Leu | 1.96 |
| Tyr | 0.91 |
| Phe | 1.25 |
| Lys | 1.91 |
| His | 0.64 |
| Arg | 7.45 |
| Pro | 0.74 |
| Total amino acids | 52.17 |

As a result, as shown in Table 1, the total amino acid content based on dry weight of the obtained Thraustochytrid microalgal extract was confirmed as 52.17%, and it was confirmed that the content of glutamic acid was the highest, and the amino acid content was high in order of arginine (Arg) and aspartic acid (Asp).

### Example 2. Confirmation of cytotoxicity of microalgal extract

In order to confirm the effect on bovine myogenic stem cells (bMuSC) extracted from shank and sirloin of the Thraustochytrid microalgal extract, a WST-8 test was performed as follows.

Specifically, cells with the number of subcultures of 3 or 5 were seeded by 5,000 per well in a 96-well culture plate. 3 wells were seeded by each condition, and cultured under conditions of 37°C, 5% CO₂ for a day. After that, it was replaced with a new culture solution comprising 10% FBS and a Thraustochytrid extract of 0.001 g/L to 40 g/L and it was cultured for 3 days, and then WST-8 of 5µL was treated per well and additional culture was conducted for 3 hours. The metabolic activity of cells was determined by measuring absorbance (OD450) at a wavelength of 450 nm using a microplate spectrophotometer. Wells comprising the culture solution without cells were used as a blank control group, and wells untreated with the Thraustochytrid extract were used as a negative control group, and the relative cell viability was compared by comparing the OD450 value of the cells treated with the Thraustochytrid extract. The concentration of the Thraustochytrid extract showing the highest viability was named Eₘₐₓ, and the CC₅₀ value at which the cell viability was 50% of the negative control group was calculated using Quest Graph^{™} IC50 calculator. The result of measuring the cell viability according to the concentration of the Thraustochytrid extract measured through the present experiment was shown in FIG. 1.

As a result, as shown in FIG. 1, the cell proliferation of bMuSC was at a similar level to the control group when the Thraustochytrid extract was comprised in a concentration range of 0.001~2 g/L. When the Thraustochytrid extract was comprised at a concentration of 4 g/L, the highest cell viability was shown in the concentration range used in the experiment as increased by 115.8% compared to the control group, and accordingly, it was confirmed that the concentration of 4g/L was Eₘₐₓ. In addition, the CC₅₀ value was confirmed as 23.6 g/L.

In conclusion, when the Thraustochytrid extract was comprised at a concentration of 4 g/L or less in the culture solution, the toxicity to cells was not shown, and in particular, it could be seen that (bMuSC) had the excellent cell proliferation in Korean native cattle muscle cells, when the Thraustochytrid extract was comprised at the above concentration in the culture solution.

### Example 3. Confirmation of serum replacement effect of Thraustochytrid extract

In order to confirm the serum replacement effect of the Thraustochytrid extract, the following experiment was performed.

Specifically, a WST-8 test was performed to confirm what effect adding the Thraustochytrid extract at the Eₘₐₓ concentration confirmed in Example 2 had on the bMuSC cells under a culture condition with reduced fetal bovine serum (FBS) in the culture solution. Bovine muscle cells with the number of subcultures of 5, 6 or 15 were seeded by 5,000 cells per well, by 3 wells by condition in a 96-well culture plate. After culturing under conditions of 37°C, 5% CO₂ for a day, half of the culture solution was replaced with a culture solution comprising FBS at a concentration of 0% to 10% and the Thraustochytrid extract at a concentration of 0 g/L to 4 g/L, and the weight ratio of the added FBS and Thraustochytrid extract was as Table 2 below.

**[Table 2]**

| FBS concentrat ion (%) | Thraustochytri d extract (g/L) | Weight per media 500mL (g) | | FBS: Thraustochytrid extract weight ratio |
|---|---|---|---|---|
| | | FBS | Thraustochytri d extract | |
| 0 | 0 | 0 | 0 | - |
| | 2 | | 1 | - |
| | 4 | | 2 | - |
| 1 | 0 | 5 | 0 | - |
| | 2 | | 1 | 5:1 |
| | 4 | | 2 | 2.5:1 |
| 2 | 0 | 10 | 0 | - |
| | 2 | | 1 | 10:1 |
| | 4 | | 2 | 5:1 |
| 5 | 0 | 25 | 0 | - |
| | 2 | | 1 | 25:1 |
| | 4 | | 2 | 12.5:1 |
| 10 | 0 | 50 | 0 | - |
| | 2 | | 1 | 50:1 |
| | 4 | | 2 | 25:1 |

Culture was performed for a day to allow the cells to adapt to the new culture solution, and after that, it was replaced with a new culture solution once again, and the WST-8 test was performed on days 3 and 6 from the time of replacement. The cell proliferation on days 3 and 6 was determined by comparing the measured OD450 value with the OD450 value measured on day 0, and the measured result was shown in FIG. 2 and Table 3 below.

**[Table 3]**

| | Thraustochytri d extract (g/L) | Cell viability (% of D0) | |
|---|---|---|---|
| | | On day 3 (D3) | On day 6 (D6) |
| 0% FB S | 0 | 13.8 | 10.2 |
| | 2 | 12.6 | 8.0 |
| | 4 | 24.0 | 9.0 |
| 1% FBS | 0 | 26.6 | 19.6 |
| | 2 | 31.2 | 40.2 |
| | 4 | 42.7 | 24.0 |
| 2% FB S | 0 | 32.4 | 29.2 |
| | 2 | 40.7 | 59.3 |
| | 4 | 58.2 | 66.4 |
| 5% FBS | 0 | 61.3 | 63.4 |
| | 2 | 58.7 | 80.2 |
| | 4 | 74.6 | 110.3 |
| 10% FBS | 0 | 100.0 | 100.0 |
| | 2 | 88.5 | 108.4 |
| | 4 | 104.0 | 126.9 |

As a result, as shown in FIG. 2 and Table 3, it was confirmed that the cell proliferation increased, when the bMuSC cells were cultured in a culture medium comprising the Thraustochytrid extract. When the Thraustochytrid extract was comprised at the Eₘₐₓ concentration, based on the day-6 culture result, the cell proliferation rate increased by 26.9% compared to the control group untreated with the Thraustochytrid extract under the 10% FBS condition, and it increased by 74.0% under the 5% FBS condition, and increased by 127.4% under the 2% FBS condition. Under the 5% FBS condition, the cell proliferation at an equivalent or higher level compared to the 10% FBS control group was shown. Therefore, it could be confirmed that the Thraustochytrid extract could induce proliferation of bMuSC cells by replacing FBS, and specifically, it could be seen that an FBS replacement rate of 50% to 80% was shown, when the Thraustochytrid extract was comprised at the Eₘₐₓ concentration of 4 g/L.

## Claims

1. A culture medium composition for cell culture, comprising a Thraustochytrid family microalgal extract.

2. The culture medium composition for cell culture according to claim 1, wherein the Thraustochytrid family microalgal extract is comprised at a concentration of 23.6 g/L or less.

3. The culture medium composition for cell culture according to claim 1, wherein the Thraustochytrid family microalgal extract is comprised at a concentration of 0.4 g/L to 10 g/L or less.

4. The culture medium composition for cell culture according to claim 1, wherein the Thraustochytrid family microalgae is any one selected from the group consisting of *Schizochytrium* sp., *Aurantiochytrium* sp., *Thraustochytrium* sp. and *UIkenia* sp.

5. The culture medium composition for cell culture according to claim 1, wherein the Thraustochytrid family microalgal extract has a total amino acid content of 40% or more based on dry weight.

6. The culture medium composition for cell culture according to claim 1, wherein the cell is a myogenic stem cell or a muscle cell.

7. The culture medium composition for cell culture according to claim 1, wherein the cell is bovine or porcine derived cell.

8. The culture medium composition for cell culture according to claim 1, wherein the composition further comprises serum.

9. The culture medium composition for cell culture according to claim 8, wherein the serum is any one or more selected from the group consisting of fetal bovine serum (FBS), bovine calf serum (BCS), human serum and horse serum (HS).

10. The culture medium composition for cell culture according to claim 8, wherein the serum is comprised at a weight ratio of 1000: 1 to 1:1 based on the Thraustochytrid family microalgal extract (serum: Thraustochytrid family microalgal extract).

11. A culture medium additive for cell culture, comprising a Thraustochytrid family microalgal extract.

12. A method for cell culture, comprising culturing isolated cells using the culture medium composition for cell culture of any one claim of claim 1 to claim 10.
